# EUROPEAN PATENT SPECIFICATION

(11) **EP 3 958 852 B1**
(45) Date of publication and mention of the grant of the patent: **01.10.2025**
(21) Application number: 20794617.9
(22) Date of filing: 22.04.2020
(51) Int. Cl.: A61K 31/192, A61K 45/06, A61K 47/10, A61K 9/48, A61P 29/00

(54) **MINI SOFTGEL NAPROXEN COMPOSITION**
MINI-WEICHGEL-NAPROXENZUSAMMENSETZUNG
COMPOSITION DE NAPROXÈNE EN MINI-CAPSULE MOLLE

(30) Priority: 26.04.2019 US 201962839198 P
(43) Date of publication of application: 02.03.2022
(73) Proprietor: R.P. Scherer Technologies, LLC, Carson City, NV 89703 (US)
(72) Inventor: SHELLEY, Rickey, Steve, Largo, FL 33770 (US)
(74) Representative: Maiwald GmbH
(86) International application number: PCT/US2020/029234
(87) International publication number: WO 2020/219499

(56) References cited:
- US-A- 5 360 615
- US-A- 5 360 615
- US-A1- 2003 219 477
- US-A1- 2017 296 495
- AMRAN ET AL.: "The Pharmacokinetic Study of Aspirin", PARACETAMOL AND NAPROXEN WITH MAGNESIUM SULFATE, 22 May 2015 (2015-05-22), XP055757270, Retrieved from the Internet <URL:https://www.longdom.org/open-access/the-pharmacokinetic-study-of-aspirin-paracetamol-and-naproxen-with-magnesium-sulfate-2153-2435-1000372.pdf>> [retrieved on 20200629]

## Description

### RELATED APPLICATIONS

This application claims priority to United States Provisional Patent Application No. 62/839,198, filed April 26, 2019.

### FIELD OF THE INVENTION

The present invention relates to the field of mini softgel pharmaceutical compositions that comprise naproxen. The present invention is also related to methods of preparing and methods of using such pharmaceutical compositions.

### BACKGROUND OF THE INVENTION

Soft gelatin capsules (referred to as liquid gels or softgels) are a unique drug delivery system that can provide distinct advantages over traditional dosage forms such as tablets, hard gelatin capsules and liquids. Softgel is a hermetically sealed, one-piece capsule with a liquid or semisolid fill. The softgel includes two major components, the shell composition and the fill composition. Exemplary shell composition may include gelatin, plasticizer and water. The fill compositions can include a wide variety of vehicles and can either be a solution or a suspension.

Soft gelatin capsules may be favored by many people for oral administration of medications, at least in part because of their ease of swallowing. The soft gelatin shell makes the capsule easier to swallow, especially for the elderly, than a tablet or a hard capsule. It is possible to make the softgel capsules even easier to swallow by reducing their size. To do so, it may be advantageous to optimize the amount of active pharmaceutical ingredient and solvent so as to achieve a highly concentrated solution in the fill composition. A greater dose may be achieved with a more concentrated fill composition without compromising the size of the softgel and its ease of use. US 5,360, 615 B relates to a solvent system enhancing the solubility of highly concentrated solutions of acidic, basic and amphoteric pharmaceuticals suitable for softgel filling.

### OBJECTS AND SUMMARY OF THE INVENTION

Embodiments of the present disclosure may be directed to a mini softgel pharmaceutical composition that is stable and exhibits target pharmacokinetic parameters, and a method of preparing said composition. The scope of the invention is defined by the appended claims.

The fill composition comprises a reaction product of naproxen free acid and potassium hydroxide. The molar ratio of potassium hydroxide to naproxen free acid in the reaction is less than 1 (+/-5%). The softgel has a size of 8 to 14, or of 10 to 12. The fill composition may have a weight of from 200mg (+/-5%) to 800mg (+/-5%), of from 400mg (+/-5%) to 800mg (+/-5%), or of from 600mg (+/-5%) to 700mg (+/-5%). The pharmaceutical composition may be a mini softgel shaped in an oblong shape or in an oval shape. The fill composition comprises naproxen potassium salt at a concentration of from 65 wt% to about 75 wt%, based on the total combined weight of naproxen free acid and naproxen potassium salt.

In certain embodiments the target pharmacokinetic parameters may include mean Tₘₐₓ, mean Cₘₐₓ, and mean AUC. The pharmacokinetic values may be with respect to administration to a population of subj ects, in a fed or in a fasted state, of a 200 mg dose of naproxen free acid or pharmaceutically acceptable salt thereof. In certain embodiments, the mean Tₘₐₓ (in a fed state) may range from about 1 hours to about 6 hours, or from about 2 hours to about 5 hours. In certain embodiments, the mean Cₘₐₓ (in a fed state) may range from about 20µg/mL to about 55µg/mL, or from about 25µg/mL to about 50µg/mL. In certain embodiments, the AUC (in a fed state) may range from about 400µg·hr/mL to about 1000µg·hr/mL, or from about 500µg·hr/mL to about 950µg·hr/mL. In certain embodiments, the mean Tₘₐₓ (in a fasted state) may range from about 0.5 hours to about 4 hours, or from about 1 hours to about 2.5 hours. In certain embodiments, the mean Cₘₐₓ (in a fasted state) may range from about 25µg/mL to about 70µg/mL, or from about 30µg/mL to about 60µg/mL. In certain embodiments, the AUC (in a fasted state) may range from about 450µg·hr/mL to about 1000µg·hr/mL, or from about 500µg·hr/mL to about 975µg·hr/mL.

The present disclosure is directed to a method of preparing any of the pharmaceutical compositions disclosed herein by incorporating naproxen free acid and potassium hydroxide in a mini softgel capsule.

### DEFINITIONS

As used herein, the singular forms "a," "an," and "the" include plural references unless the context clearly indicates otherwise. Thus, for example, reference to "an active pharmaceutical ingredient" includes a single active pharmaceutical ingredient as well as a mixture of two or more different active pharmaceutical ingredients, and reference to an "excipient" includes a single excipient as well as a mixture of two or more different excipients, and the like.

As used herein, the term "about" in connection with a measured quantity, refers to the recited number ±5%, such that "about 10" would include from 9.5 to 10.5.

As used herein, the terms "active agent," "active ingredient," "active pharmaceutical ingredient," "API," and "drug" refer to any material that is intended to produce a therapeutic, prophylactic, or other intended effect, whether or not approved by a government agency for that purpose. These terms with respect to specific agents include all pharmaceutically active agents, all pharmaceutically acceptable salts thereof, complexes, stereoisomers, crystalline forms, co-crystals, ether, esters, hydrates, solvates, and mixtures thereof, where the form is pharmaceutically active.

As used herein, the term "stereoisomers" is a general term for all isomers of individual molecules that differ only in the orientation of their atoms in space. It includes enantiomers and isomers of compounds with one or more chiral centers that are not mirror images of one another (diastereomers).

The term "enantiomer" or "enantiomeric" refers to a molecule that is nonsuperimposable on its mirror image and hence optically active wherein the enantiomer rotates the plane of polarized light in one direction by a certain degree, and its mirror image rotates the plane of polarized light by the same degree but in the opposite direction.

The term "chiral center" refers to a carbon atom to which four different groups are attached.

The term "patient" refers to a subject, an animal or a human, who has presented a clinical manifestation of a particular symptom or symptoms suggesting the need for treatment, who is treated preventatively or prophylactically for a condition, or who has been diagnosed with a condition to be treated. The term "subject" is inclusive of the definition of the term "patient" and does not exclude individuals who are otherwise healthy.

"Pharmaceutically acceptable salts" include, but are not limited to, inorganic acid salts such as hydrochloride, hydrobromide, sulfate, phosphate and the like; organic acid salts such as formate, acetate, trifluoroacetate, maleate, tartrate and the like; sulfonates such as methanesulfonate, benzenesulfonate, p-toluenesulfonate and the like; amino acid salts such as arginate, asparaginate, glutamate and the like; metal salts such as sodium salt, potassium salt, cesium salt and the like; alkaline earth metals such as calcium salt, magnesium salt and the like; and organic amine salts such as triethylamine salt, pyridine salt, picoline salt, ethanolamine salt, triethanolamine salt, discyclohexylamine salt, N,N'-dibenzylethylenediamine salt and the like.

Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to illuminate certain materials and methods and does not pose a limitation on scope. No language in the specification should be construed as indicating any non-claimed element as essential to the practice of the disclosed materials and methods.

The term "condition" or "conditions" refers to those medical conditions that can be treated or prevented by administration to a subject of an effective amount of an active agent, e.g., pain.

The terms "treatment of" and "treating" includes the lessening of the severity of or cessation of a condition or lessening the severity of or cessation of symptoms of a condition.

The terms "prevention of" and "preventing" includes the avoidance of the onset of a condition.

"Therapeutically effective amount" is intended to include an amount of an active agent, or an amount of the combination of active agents, e.g., to treat or prevent the condition, or to treat the symptoms of the condition, in a subject.

The phrase "pharmaceutically acceptable" refers to those compounds, materials, compositions, and/or dosage forms that are, within the scope of sound medical judgment, suitable for use in contact with the tissues of human beings and animals without excessive toxicity, irritation, allergic response, or other problems or complications commensurate with a reasonable benefit/risk ratio.

### DETAILED DESCRIPTION

The present invention relates to a pharmaceutical composition comprising a softgel and a fill composition.

The API in the fill compositions disclosed herein are naproxen free acid and the pharmaceutically acceptable potassium salt thereof. The potassium salt form of the API is present in the fill composition from about 65wt% to about 75wt%, to about 70wt%, about 66wt%, about 67wt%, about 68wt%, about 69wt%, about 70wt%, about 71wt%, about 72wt%, about 73wt%, about 74wt%, or about 75wt%, or any sub-range, or single concentration value therein, calculated based on the total combined weight of the free acid form of the API (e.g., naproxen free acid) and the potassium salt form of the API (e.g., naproxen salt).

The above ranges are advantageous for attaining a stable softgel naproxen composition that exhibits the target pharmacokinetic parameters described herein. If a softgel pharmaceutical composition comprises more than about 75wt% of naproxen potassium salt (based on the total combined weight of naproxen free acid and naproxen potassium salt in the fill composition), the softgel may be unstable and melt at accelerated conditions. On the other hand, if a softgel pharmaceutical composition comprises less than about 55wt% of naproxen potassium salt (based on the total combined weight of naproxen free acid and naproxen potassium salt in the fill composition), the softgel may not be as efficacious in treating and/or preventing a target condition (i.e., it may not exhibit the pharmacokinetic performance described below in further detail).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 1 month at 40 °C and 75% relative humidity (RH).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 2 month at 40 °C and 75% relative humidity (RH).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 3 month at 40 °C and 75% relative humidity (RH).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 6 month at 40 °C and 75% relative humidity (RH).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 9 month at 40 °C and 75% relative humidity (RH).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 1 month at 30 °C and 65% relative humidity (RH).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 2 month at 30 °C and 65% relative humidity (RH).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 3 month at 30 °C and 65% relative humidity (RH).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 6 month at 30 °C and 65% relative humidity (RH).

In certain embodiments, the fill composition maintains at least about 90%, at least about 95%, at least about 98%, or at least about 99% of the amount of naproxen or pharmaceutically acceptable salt thereof (e.g., naproxen free acid or potassium salt of naproxen) after storage for 9 month at 30 °C and 65% relative humidity (RH).

The base in the fill compositions disclosed herein may be selected due to its strength and its interaction with the API. For instance, it may be advantageous to select a base that is strong enough to convert (and maintain) a majority of an API's free acid form (e.g., naproxen free acid) to its salt form (e.g., naproxen salt). Furthermore, it may be advantageous to select a base that enhances the solubility of an API's salt form (e.g., naproxen salt) and an API's free acid form (e.g., naproxen free acid), when combined. Such enhanced solubility may be useful to form concentrated solutions of the API and a pharmaceutically acceptable salt thereof. Such concentrated solutions may be useful in forming high dosage pharmaceutical compositions in dosage forms having smaller sizes (i.e., a greater amount of API in a lesser volume).

The base is potassium hydroxide. Potassium hydroxide is used to neutralize the free acid form of naproxen and to form the potassium salt form of naproxen. Potassium hydroxide may be useful in naproxen pharmaceutical compositions since the potassium salt of naproxen has better solubility in low molecular weight polyethylene glycol solvents as compared to certain other salts of naproxen (e.g., sodium salt of naproxen). Additionally, the combination of free acid of naproxen and potassium salt of naproxen is more soluble in low molecular weight polyethylene glycol than each of the components individually. Thus, by combining the potassium salt of naproxen and the free acid form of naproxen in the fill composition, it is possible to attain a pharmaceutical composition that has higher concentrations of naproxen API or a pharmaceutically acceptable salt thereof (as compared to pharmaceutical compositions lacking free acid of naproxen or comprising a different salt form of naproxen).

The size and shape of the final softgel capsule can vary. The shape of the capsule may be, but is not limited to, round, oval, oblong, or a non-standard shape. Typical softgel capsule shapes and sizes may be, but are not limited to, those as shown in Table 1 below.

**Table 1 - Nominal Soft Gelatin Capsule Shapes and Their Approximate Sizes**

| | Oblong Shape | | Oval Shape | |
|---|---|---|---|---|
| Nominal Size | Minimum Volume (ml) | Maximum Volume (ml) | Minimum Volume (ml) | Maximum Volume (ml) |
| 1 | 0.03 | 0.08 | 0.03 | 0.06 |
| 2 | 0.08 | 0.14 | 0.06 | 0.09 |
| 3 | 0.14 | 0.20 | 0.11 | 0.17 |
| 4 | 0.20 | 0.30 | 0.15 | 0.22 |
| 5 | 0.26 | 0.37 | 0.23 | 0.30 |
| 6 | 0.32 | 0.46 | 0.26 | 0.38 |
| 8 | 0.43 | 0.63 | 0.33 | 0.48 |
| 10 | 0.53 | 0.76 | 0.42 | 0.60 |
| 12 | 0.64 | 0.93 | 0.50 | 0.73 |
| 14 | 0.75 | 1.06 | 0.60 | 0.85 |
| 16 | 0.85 | 1.23 | 0.70 | 1.00 |
| 18 | 0.96 | 1.40 | 0.76 | 1.10 |
| 20 | 1.10 | 1.55 | 0.85 | 1.20 |
| 22 | 1.20 | 1.70 | 0.95 | 1.35 |
| 24 | 1.30 | 1.85 | 1.05 | 1.46 |
| 26 | 1.40 | 2.00 | 1.13 | 1.60 |
| 28 | 1.50 | 2.15 | 1.23 | 1.70 |
| 30 | 1.65 | 2.30 | 1.30 | 1.85 |

As indicated above, concentrated solutions allow for the inclusion of high dosages in a smaller volume and in response in a smaller softgel capsule size. The softgel capsule sizes disclosed here have a size ranging from 8 to 14, from 10 to 12, and all sizes in between, such as, 8, 9, 10, 11, 12, 13, or 14. In certain embodiments, the softgel capsule sizes disclosed herein may be about 20% to about 30% smaller than an existing equivalent dosage form (e.g., about 20% to about 30% smaller than Aleve^{®} liquid-gel Naproxen Sodium 220 mg).

In some embodiments, the fill compositions disclosed herein may have a weight of from about 200mg to about 800mg, from about 300mg to about 750mg, from about 400mg to about 700mg, from about 400mg to about 800mg, about 200 mg, about 250mg, about 300mg, about 350mg, about 400mg, about 450mg, about 500mg, about 550mg, about 600mg, about 610mg, about 620mg, about 630mg, about 640mg, about 650mg, about 660mg, about 670mg, about 680mg, about 690mg, about 700mg, about 750mg, or about 800mg, or any sub-range, or single weight value therein.

In certain embodiments, the molar ratio of the base (being potassium hydroxide) to the free acid form of the API (e.g., naproxen free acid) is less than about 1. For instance, the molar ratio of the base (being potassium hydroxide) to the API (e.g., naproxen free acid) may be from about 0.55 to about 0.75, from about 0.6 to about 0.7, about 0.55, about 0.56, about 0.57, about 0.58, about 0.59, about 0.60, about 0.61, about 0.62, about 0.63, about 0.64, about 0.65, about 0.66, about 0.67, about 0.68, about 0.69, about 0.70, about 0.71, about 0.72, about 0.73, about 0.74, or about 0.75, or any sub-range, or single molar ratio value therein.

If a softgel pharmaceutical composition comprises a molar ratio of base (such as potassium hydroxide) to API (such as naproxen free acid) of more than about 0.75, the pH and API salt (e.g., naproxen salt) content of the fill composition may be too high, resulting in an unstable softgel capsule. On the other hand, if a softgel pharmaceutical composition comprises a molar ratio of base (such as potassium hydroxide) to API (such as naproxen free acid) of less than about 0.55, the API salt (such as naproxen salt) content of the fill composition may be too low and the pharmaceutical composition may not be as efficacious in treating and/or preventing a target condition (i.e., it may not exhibit the pharmacokinetic performance described below in further detail).

The pharmaceutical composition may comprise naproxen free acid and naproxen potassium salt. The reaction product of naproxen free acid and potassium hydroxide is the potassium salt of naproxen as shown in the chemical reaction depicted below. As such, the amount of potassium salt of naproxen may relate to the amount of naproxen free acid and potassium hydroxide in the fill composition.

**Naproxen Free Acid + KOH → Potassium Salt of Naproxen + H₂O**

In some embodiments, the pharmaceutical compositions disclosed herein may be efficacious in treating a target condition (such as pain). The API (e.g., naproxen or a pharmaceutically acceptable salt thereof) may be present in the pharmaceutical composition in a therapeutically effective amount such that the pharmaceutical composition may exhibit target pharmacokinetic parameters for a population of subjects (e.g., mean Tₘₐₓ, mean Cₘₐₓ, and/or mean AUC) upon oral administration in a fed state and/or in a fasted state.

For instance, pharmaceutical compositions disclosed herein may provide a mean Tₘₐₓ ranging from about 1 hour to about 6 hours, or from about 2 hours to about 5 hours upon oral administration to a population of subjects in a fed state of a 200 mg dose of naproxen free acid or a pharmaceutically acceptable salt thereof.

For instance, pharmaceutical compositions disclosed herein may provide a mean Tₘₐₓ ranging from about 0.5 hour to about 4 hours, or from about 1 hour to about 2.5 hours upon oral administration to a population of subjects in a fasted state of a 200 mg dose of naproxen free acid or a pharmaceutically acceptable salt thereof.

For instance, pharmaceutical compositions disclosed herein may provide a mean Cₘₐₓ ranging from about 20 µg/mL to about 55 µg/mL, or from about 25 µg/mL to about 50 µg/mL upon oral administration to a population of subjects in a fed state, based on a dose of 200 mg dose of naproxen free acid or a pharmaceutically acceptable salt thereof.

For instance, pharmaceutical compositions disclosed herein may provide a mean Cₘₐₓ ranging from about 25 µg/mL to about 70 µg/mL, or from about 30 µg/mL to about 60 µg/mL upon oral administration to a population of subjects in a fasted state, based on a dose of 200 mg dose of naproxen free acid or a pharmaceutically acceptable salt thereof.

For instance, pharmaceutical compositions disclosed herein may provide a mean AUC ranging from about 400 µg·hr/mL to about 1000 µg·hr/mL, or from about 500 µg·hr/mL to about 950 µg·hr/mL upon oral administration to a population of subjects in a fed state, based on a dose of 200 mg dose of naproxen free acid or a pharmaceutically acceptable salt thereof.

For instance, pharmaceutical compositions disclosed herein may provide a mean AUC ranging from about 450 µg·hr/mL to about 1000 µg·hr/mL, or from about 500 µg·hr/mL to about 975 µg·hr/mL upon oral administration to a population of subjects in a fasted state, based on a dose of 200 mg dose of naproxen free acid or a pharmaceutically acceptable salt thereof.

Pharmaceutical compositions disclosed herein may further comprise a pharmaceutically acceptable solvent. A suitable solvent may be one that dissolves other components of the pharmaceutical composition to obtain a clear solution in a liquid form. Exemplary solvents may include, without limitations, low molecular weight polyethylene glycol. The polyethylene glycol may have an average molecular weight ranging from about 200 to about 800 daltons, from about 400 to about 700 daltons, or about 600 daltons, or any sub-range or single value of molecular weight range of polyethylene glycol therein. Other exemplary liquid polyethylene glycol may be include, without limitations, PEG 200, PEG 300, PEG 400, PEG 600, PEG 800, and combinations thereof.

In certain embodiments, the pharmaceutical compositions disclosed herein may consist essentially of (or consist of) a softgel and a fill composition. The fill composition may consist essentially of (or consist of) a reaction product of naproxen free acid or a pharmaceutically acceptable salt thereof and potassium hydroxide. The fill composition may, in addition to the reaction product, further consist essentially of (or consist of) a solvent (such as polyethylene glycol and/or water). The softgel capsule's size, the amount of the components, and the pharmacokinetic characteristics of the pharmaceutical composition may include any of those described herein.

Pharmaceutical compositions disclosed herein may include additional constituents/excipients, such as, surfactants (with an HLB value of less than 10 or more than 10), solvents, cosolvents, solid high molecular weight polyethylene glycol, water-soluble polymers, flavoring agents, pH modifiers, disintegrants, plasticizers, colorants, pore formers, dispersing agents, water-soluble polymers, water, glycerin, sorbitol, cyclodextrins, solubility enhancers, bioavailability enhancers, opacifying agents, enzymes, preservatives, stabilizers, antioxidants, extenders, and combinations thereof. Suitable excipients may be in a liquid, semi-solid, and/or solid form.

A surfactant with an HLB value of less than 10 may include, without limitations, ethylene oxide/propylene oxide (EO/PO) copolymers, glycerol monocaprylate, glycerol monocaprate, glycerol caprylate/caprate, glycerol monooleate, glycerol monostearate, glycerol laurate, glycerol monolinoleate, glycerol behenate, glycerol palmitostearate, petroleum and lanolin alcohols, polyoxyethylene alkyl ethers (e.g., polyoxyl 4 lauryl ether, polyoxyl 2 cetyl ether, polyoxyl 2 stearyl ether, polyoxyl 2 oleyl ether), sorbitan fatty acid esters (e.g., sorbitan monoisostearate, sorbitan monolaurate, sorbitan monopalmitate, sorbitan monostearate, sorbitan sesquiisostearate, sorbitan sesquioleate, sorbitan sesquistearate, sorbitan diisostearate, sorbitan dioleate, sorbitan triisostearate, sorbitan trioleate, sorbitan tristearate), sucrose esters, poly(ethylene glycol)-block-poly(propylene glycol)-block-poly(ethylene glycol) (pluronic copolymers), PEG-30 dipolyhydroxystearate, propylene glycol monocaprylate, propylene glycol dilaurate, propylene glycol monolaurate, propylene glycol monostearate, propylene glycol isostearate, sorbitan monooleate, sorbitan monostearate, sorbitan monopalmitate, sorbitan tristearate, sorbitan trioleate, and combinations thereof.

Suitable surfactant with an HLB value of greater than 10 may include, without limitations polysorbate 80-polyoxyethylene (20) sorbitan monooleate, polyoxyl 40 hydrogenated castor oil, polyoxyl 35 castor oil, caprylocaproyl macrogol glycerides, and combinations thereof.

Exemplary solvents and/or cosolvents may include, without limitations, ethanol, propylene glycol, glycerin, polyethylene glycol, and combinations thereof.

Exemplary solid high molecular weight polyethylene glycol may include, without limitations, PEG 3350, PEG 4000, PEG 4600, PEG 5000, PEG 6000, PEG 7000, PEG 8000, PEG of up to 10000, and combinations thereof.

Exemplary water-soluble polymers may include, without limitations, hydroxypropylmethylcellulose (HPMC), hydroxypropylcellulose (HPC), hydroxyethylcellulose (HEC), gums, and combinations thereof.

Exemplary plasticizers may include, without limitations, sugar alcohol plasticizer such as isomalt, maltitol, sorbitol, xylitol, erythritol, adonitol, dulcitol, pentaerythritol, or mannitol; or polyol plasticizer such as glycerin, diglycerin, ethylene glycol, diethylene glycol, triethyleneglycol, tetraethylene glycol, dipropylene glycol, a polyethylene glycol up to 10,000 MW, neopentyl glycol, propylene glycol, 1,3-propanediol, 2-methyl-1,3-propanediol, trimethylolpropane, a polyether polyol, ethanol amines, low molecular weight polymers, oligomers, copolymers, oils, small organic molecules, low molecular weight polyols having aliphatic hydroxyls, ester-type plasticizers, glycol ethers, poly(propylene glycol), multi-block polymers, single block polymers, citrate ester-type plasticizers, triacetin, 1,2-butylene glycol, 2,3-butylene glycol, styrene glycol, monopropylene glycol monoisopropyl ether, propylene glycol monoethyl ether, ethylene glycol monoethyl ether, diethylene glycol monoethyl ether, sorbitol lactate, ethyl lactate, butyl lactate, ethyl glycolate, dibutyl sebacate, acetyltributylcitrate, triethyl citrate, glyceryl monostearate, polysorbate 80, acetyl triethyl citrate, tributyl citrate and allyl glycolate, and mixtures thereof.

Exemplary colorants may include, without limitations, colors such as e.g., white, black, yellow, blue, green, pink, red, orange, violet, indigo, and brown.

Exemplary flavoring agents may include, without limitations, breath freshening compounds like menthol, spearmint, and cinnamon, coffee beans, other flavors or fragrances such as fruit flavors (e.g., cherry, orange, grape, etc.), especially those used for oral hygiene, as well as actives used in dental and oral cleansing such as quaternary ammonium bases. The effect of flavors may be enhanced using flavor enhancers like tartaric acid, citric acid, vanillin, or the like.

Exemplary sweeteners may include, without limitations, one or more artificial sweeteners, one or more natural sweeteners, or a combination thereof. Artificial sweeteners include, e.g., acesulfame and its various salts such as the potassium salt (available as Sunett^{®}), alitame, aspartame (available as NutraSweet^{®} and Equal^{®}), salt of aspartame-acesulfame (available as Twinsweet^{®}), neohesperidin dihydrochalcone, naringin dihydrochalcone, dihydrochalcone compounds, neotame, sodium cyclamate, saccharin and its various salts such as the sodium salt (available as Sweet'N Low^{®}), stevia, chloro derivatives of sucrose such as sucralose (available as Kaltame^{®} and Splenda^{®}), and mogrosides. Natural sweeteners include, e.g., glucose, dextrose, invert sugar, fructose, sucrose, glycyrrhizin; monoammonium glycyrrhizinate (sold under the trade name MagnaSweet^{®}); Stevia rebaudiana (Stevioside), natural intensive sweeteners, such as Lo Han Kuo, polyols such as sorbitol, mannitol, xylitol, erythritol, and the like.

Exemplary pH modifiers may include, without limitations, hydrochloric acid, potassium hydroxide, sodium hydroxide, ammonium hydroxide, sulfuric acid, phosphoric acid, and nitric acid.

Other exemplary excipients that may be in the pharmaceutical composition may include, but not be limited to, gelatin, water-soluble polysaccharides (such as alginates, carrageenans, guar gum, agar-agar, xanthan gum, gellan gum, gum arabic and related gums (gum ghatti, gum karaya, gum tragancanth), pectin), water-soluble derivatives of cellulose (such as alkylcelluloses, hydroxyalkylcelluloses, and hydroxyalkylalkylcelluloses (such as methylcelulose, hydroxymethylcellulose, hydroxyethylcellulose, hydroxypropylcellulose, hydroxyethylmethylcellulose, hydroxypropylmethylcellulose, hydroxybutylmethylcellulose), cellulose esters and hydroxyalkylcellulose esters (such as cellulose acetate phthalate (CAP)), hydroxypropylmethylcellulose (HPMC), carboxyalkylcelluloses, carboxyalkylalkylcelluloses, carboxyalkylcellulose esters (such as carboxymethylcellulose and their alkali metal salts)), water-soluble synthetic polymers (such as polyacrylic acids, polyacrylamides, and polyacrylic acid esters, polymethacrylic acids, polymethacrylamides, and polymethacrylic acid esters), polyvinylacetates, polyvinylalcohols, polyvinylacetatephthalates (PVAP), polyvinylpyrrolidone (PVP), PVY/vinyl acetate copolymer.

In some embodiments, the excipients may be present in the pharmaceutical/fill composition at a concentration of about 20 wt% or less, about 15 wt% or less, about 10 wt% or less, about 5 wt% or less, about 4 wt% or less, about 3 wt% or less, about 2 wt% or less, about 1 wt% or less, about 0.5 wt% or less, about 0.1 wt% or less based on the total weight of the pharmaceutical/fill composition. In some embodiments, the fill composition may have no excipients other than a polyethylene glycol solvent (e.g., about 0 wt% of excipient other than polyethylene glycol solvent). In some embodiments, the pharmaceutical/fill composition may comprise excipients in an amount ranging, e.g., from about 2 wt% to about 50 wt%, from about 6 wt% to about 40 wt%, from about 10 wt% to about 30 wt%, from about 10 wt% to about 40 wt%, from about 15 wt% to about 35 wt%, from about 20 wt% to about 30 wt%, from about 20 wt% to about 25 wt%, or from about 15 wt% to about 25 wt%, based on the total weight of the pharmaceutical/fill composition.

In some embodiments, the API (in its free acid form and/or in its salt form) may be present in the fill composition at a concentration of about 15 wt% to about 40 wt%, about 20 wt% to about 30 wt%, about 15 wt%, about 16 wt%, about 17 wt%, about 18 wt%, about 19 wt%, about 20 wt%, about 21 wt%, about 22 wt%, about 23 wt%, about 24 wt%, about 25 wt%, about 26 wt%, about 27 wt%, about 28 wt%, about 29 wt%, about 30 wt%, about 31 wt%, about 32 wt%, about 33 wt%, about 34 wt%, about 35 wt%, about 36 wt%, about 37 wt%, about 38 wt%, about 39 wt%, or about 40 wt%, based on total weight of the fill composition.

In some embodiment, the fill composition may have a moisture content of up to about 15%, up to about 12%, up to about 10%, up to about 9%, up to about 8%, up to about 7%, up to about 6%, up to about 5%, up to about 4%, up to about 3%, up to about 2%, up to about 1%, or free of water altogether (i.e., moisture content of 0%). In certain embodiments, a moisture content greater than such upper ranges may result in a softgel formulation that is not physically stable at certain conditions (such as 40 °C and 75% relative humidity).

In some embodiments, the softgel compositions disclosed herein may have a hardness of about 2 N to about 20 N, of about 4N to about 15 N, of about 6 N to about 13N, of about 8 N to about 12 N, or any sub-range or single value of hardness encompassed therein.

The hardness and/or moisture content of the dosage forms disclosed herein may be attained by drying the softgel formulation for a duration of up to about 20 days, about 1 day to about 18 days, about 5 days to about 15 days, about 7 days to about 13 days, or about 10 days to about 12 days, or any sub-range or single duration value encompassed therein.

Any of the other excipients disclosed herein may be incorporated into the dosage form at various steps in any of the methods disclosed herein.

### ILLUSTRATIVE EXAMPLE

The following examples are set forth to assist in understanding the invention and should not be construed as specifically limiting the invention described and claimed herein.

### Example 1: Naproxen Mini Softgel Formulation

Table 2 below depicts six fill compositions prepared by neutralizing naproxen free acid with potassium hydroxide at different mole ratios and different fill weights.

**Table 2 - Naproxen Mini Softgel Formulations**

| **Ingredients** / **Sample** | **1*)** | **2 *)** | **3** | **4** | **5** | **6** |
|---|---|---|---|---|---|---|
| Expected potassium salt of naproxen (wt% based on total combined weight of potassium salt of naproxen and naproxen free acid) | 60 | 62.5 | 65 | 67.5 | 65 | 65 |
| PEG600 | 420.46 | 355.89 | 415.33 | 350.77 | 416.2 | 354.2 |
| Naproxen Free Acid | 200 | 200 | 200 | 200 | 200 | 200 |
| Water | 28.16 | 29.34 | 30.51 | 31.68 | 30.1 | 30.1 |
| KOH | 33.38 | 34.77 | 36.16 | 37.55 | 35.7 | 35.7 |
| Total (g) | 682 | 620 | 682 | 620 | 682 | 620 |

| | | | | | | |
|---|---|---|---|---|---|---|
| *) **comparative samples** | | | | | | |

Table 3 below depicts three softgel formulas used for encompassing the fill compositions described in Table 2.

**Table 3 - Naproxen Reduced Fill - Gel Formulas**

| **Ingredients** / **Sample** | **1 *)** | **2*)** | 3 | 4 | 5 | 6 |
|---|---|---|---|---|---|---|
| Gelatin (Type RS) (Type B) | 43.30 | | -- | | -- | |
| Gelatin (Type 170) | -- | | 47.00 | | -- | |
| Gelatin (Type 150) | -- | | -- | | 40.70 | |
| Sorbitol Sorbitan Solution | 24.70 | | 24.44 | | 11.89 | |
| Purified Water | 32.00 | | 28.56 | | 32.61 | |
| Glycerin, Natural 96% | -- | | -- | | 40.70 | |

| | | | | | | |
|---|---|---|---|---|---|---|
| *) **comparative samples** | | | | | | |

The naproxen mini softgel formulations from Tables 2 and 3 were prepared according to the following process:
Deionized water was added to an approximately 151 liters (40 gallon) closed mixing vessel with bottom mixer. The water was cooled to approximately 15 degrees C. Full vacuum was obtained on the vessel and while mixing at approximately 100 RPM, Potassium Hydroxide was slowly vacuum transferred into the vessel maintaining the temperature at less than 40 degrees C. The Potassium Hydroxide/water solution was mixed until the potassium hydroxide was completely dissolved. The temperature of the solution was maintained between 20 - 25 degrees C.

Polyethylene Glycol 600 was vacuum transferred into a separate, approximately 1136 liters (300 gallon) closed mixing vessel. The jacket of the closed vessel was set at 27 degrees C. While mixing under vacuum with the disperser set at 1200 RPM and the sweep set at 20 RPM, approximately half of the Naproxen free acid was vacuum transferred into the closed mixing vessel, containing the polyethylene glycol 600, and mixed until homogeneous while still under vacuum.

After completion of mixing, approximately half of the Potassium Hydroxide solution was slowly vacuum transferred into the 1136 liters (300 gallon) closed mixing tank while mixing with disperser at 1200 RPM and sweep at 20 RPM. The temperature was kept at below about 40 degrees C.

After mixing for approximately 10 minutes, the remainder of the Naproxen free acid was vacuum transferred into the closed 1136 liters (300 gallon) mixing tank and mixed until homogenous.

Following the Naproxen free acid transfer, the remainder of the Potassium Hydroxide was vacuum transferred into the closed 300 gallon mixing tank maintaining the temperature at less than 40 degrees C.

The mixture was mixed for at least about 20 minutes until all the Naproxen free acid was completely dissolved.

The fill solution was transferred into a closed receiver and the receiver was transferred to a rotary die encapsulation machine.

The fill material was encapsulated into a 10 oval die using a fast drying gel formula.

The softgels were dried to a fill moisture of up to about 7%. The drying time, fill moisture, and hardness for each sample are summarized in Table 4 below.

**Table 4 - Drying Results for Samples 1-6**

| **Formulation** | **Drying Time (Days)** | **Fill Moisture (%)** | **Hardness (N)** |
|---|---|---|---|
| Sample 1*) | 1 | 12.97 | 5.0 |
| | 2 | 12.64 | 6.0 |
| *Approximately six hours out of dryer, moisture=13.44% | 3 | 12.58 | 6.8 |
| | 6 | 11.44 | 7.4 |
| | 7 | 11.09 | 8.4 |
| | 8 | 10.67 | 9.0 |
| | 13 | Equipment failure | 10.2 |
| | 14 | 9.74 | 10.2 |
| | 15 | 9.60 | 10.8 |
| | 16 | 9.04 | 11.0 |
| Sample 2*) | 1 | 13.26 | 3.9 |
| *Approximately six hours out of dryer, moisture=14.06% | 2 | 12.70 | 5.1 |
| | 3 | 12.11 | 6.0 |
| | 6 | 11.17 | 7.5 |
| | 7 | 10.93 | 8.0 |
| | 8 | 10.42 | 8.7 |
| | 13 | Equipment Failure | 10.1 |
| | 14 | 9.56 | 10.2 |
| | 15 | 9.42 | 10.8 |
| | 16 | 8.71 | 10.8 |
| Sample 3 | 1 | 13.42 | 4.3 |
| *Approximately six hours out of dryer, moisture=13.44% | 2 | 12.90 | 5.1 |
| | 5 | 11.56 | 6.6 |
| | 6 | 11.27 | 6.9 |
| | 7 | 11.11 | 8.0 |
| | 12 | Equipment Failure | 9.0 |
| | 13 | 10.04 | 8.8 |
| | 14 | 9.92 | 9.5 |
| | 15 | 9.36 | 9.9 |
| Sample 4 | 1 | 13.91 | 4.2 |
| *Approximately six hours out of dryer, moisture=14.25% | 2 | 13.01 | 5.1 |
| | 5 | 11.51 | 5.9 |
| | 6 | 11.29 | 6.8 |
| | 7 | 11.12 | 7.7 |
| | 12 | Equipment Failure | 9.0 |
| | 13 | 10.01 | 8.7 |
| | 14 | 10.02 | 9.5 |
| | 15 | 9.32 | 9.8 |
| Sample 5 | 3 | 9.06 | 5.2 |
| | 4 | 8.37 | 7.1 |
| | 5 | 7.72 | 8.0 |
| | 6 | 7.42 | 8.6 |
| | 7 | 7.19 | 8.7 |
| | 10 | 6.54 | 9.8 |
| Sample 6 | 3 | 8.80 | 4.8 |
| | 4 | 8.16 | 6.9 |
| | 5 | 7.62 | 7.9 |
| | 6 | 7.36 | 8.7 |
| | 7 | 7.04 | 8.6 |
| | 10 | 6.32 | 9.7 |

| | | | |
|---|---|---|---|
| ***) comparative samples** | | | |

### Example 2: Naproxen Mini Softgel Formulation - Stability Study

Two batches of 200 mg naproxen free acid (formulations 5 and 6 from tables 2 and 3 of Example 1) were subjected to a physical and chemical stability study. The results for the first batch were established based on the following stability pull points: initial, 1 month, 2 months, and 3 months. The results are summarized in Table 5 below. The results for the second batch were established based on the following stability pull points: initial, 1 month, 2 months, 3 months, 6 months, and 9 months. The results are summarized in Table 6 below.

**Table 5 - Naproxen Mini Softgel Formulation - Stability Study Results - Batch 1**

| **Time point** | **Storage Condition** | **Method** | **Assav%** | **Related Substances** |
|---|---|---|---|---|
| T=0 | N/A | 1.1.6.211 | 98.2 | Glycerin-Ester: < LOQ |
| | | | | PEG-Nap: < LOQ |
| | | | | Total: < LOQ |
| T=1 Month | 40 °C/75% RH | 1.1.6.211 | 98.7 | Glycerin-Ester: < LOQ |
| | | | | PEG-Nap: < 0.14% |
| | | | | Total: < 0.14% |
| T=2 Month | 40 °C/75% RH | 1.1.6.211 | 98.9 | Glycerin-Ester: 0.15% |
| | | | | PEG-Nap: 0.26% |
| | | | | Total: 0.41% |
| T=3 Month | 40 °C/75% RH | 1.1.6.211 | 51.0¹ | Glycerin-Ester: < 0.21% |
| | | | | PEG-Nap: < 0.36% |
| | | | | Total: < 0.57% |

| | | | | |
|---|---|---|---|---|
| ¹ Analyst error occurred during this assay testing. There were not enough samples to repeat the assay test, therefore dissolution test was determined to be the most critical test. The dissolution result is reported in Table 7 below. | | | | |

**Table 6 - Naproxen Mini Softgel Formulation - Stability Study Results - Batch 2**

| **Time point** | **Storage Condition** | **Method** | **Assav%** | **Related Substances** |
|---|---|---|---|---|
| T=0 | N/A | 1.1.6.211 | 101.2 | Glycerin-Ester: < LOQ |
| | | | | PEG-Nap: < LOQ |
| | | | | Total: < LOQ |
| T=1 Month | 40 °C/75% RH | 1.1.6.211 | 101.6 | Glycerin-Ester: < LOQ |
| | | | | PEG-Nap: 0.15% |
| | | | | Total: 0.15% |
| T=2 Month | 40 °C/75% RH | 1.1.6.211 | 102.3 | Glycerin-Ester: 0.15% |
| | | | | PEG-Nap: 0.25% |
| | | | | Total: 0.40% |
| T=3 Month | 40 °C/75% RH | 1.1.6.211 | 51.81 | Glycerin-Ester: 0.21% |
| | | | | PEG-Nap: 0.36% |
| | | | | Total: 0.56% |
| T=6 Month | 30 °C/65% RH | 1.1.6.485 | 102.1 | Glycerin-Ester: 0.17% |
| | | | | PEG-Nap: 0.30% |
| | | | | Total: 0.46% |
| T=6 Month | 40 °C/75% RH | 1.1.6.485 | 93.7 | Glycerin-Ester: 0.46% |
| | | | | PEG-Nap: 0.62% |
| | | | | Total: 1.08% |
| T=9 Month | 30 °C/65% RH | 1.1.6.485 | 101.7 | Glycerin-Ester: 0.21% |
| | | | | PEG-Nap: 0.36% |
| | | | | Total: 0.57% |

| | | | | |
|---|---|---|---|---|
| ¹ Analyst error occurred during this assay testing as supported by results obtained at T= 6 Month and T=9 Month. There were not enough samples to repeat the assay test, therefore dissolution test was determined to be the most critical test. The dissolution result is reported in Table 8 below. | | | | |

In comparison, a comparative formulation of a commercial product, including 200 mg related active agents, displayed an assay of 104.0%, glycerin-ester at 0.11%, PEG-Nap at 0.25%, and a total of related substances at 0.36%, as analyzed based on method 1.1.6.482. The comparative formulation was purchased and tested directly from the commercial bottle.

The two batches of 200 mg naproxen free acid (for which stability study results are summarized in Tables 5 and 6 respectively) were also subjected to a dissolution test. The dissolution results for the first batch were established based on the following stability pull points: initial, 1 month, 2 months, and 3 months and are summarized in Table 7 below. The dissolution results for the second batch were established based on the following stability pull points: initial, 1 month, 2 months, 3 months, 6 months, and 9 months and are summarized in Table 8 below.

**Table 7 - Naproxen Mini Softgel Formulation - Dissolution Results - Batch 1**

| **Time point** | **Condition** | **Method** | **Dissolution** | | | | | | | | |
|---|---|---|---|---|---|---|---|---|---|---|---|
| T=0 | N/A | OTBA-THPLC-0293 | **Time (min)** | **V1** | **V2** | **V3** | **V4** | **V5** | **V6** | **Mean** | **%RSD** |
| | | | 10 | 21 | 31 | 8 | 11 | 88 | 8 | **28** | 111.4% |
| | | | 20 | 89 | 79 | 87 | 88 | 93 | 79 | **86** | 6.5% |
| | | | 35 | 95 | 93 | 95 | 95 | 96 | 97 | **95** | 1.5% |
| | | | 45 | 97 | 96 | 98 | 97 | 96 | 99 | **97** | 1.3% |
| T=1 Month | 40 °C/75% RH | 1.1.6.326 | **Time (min)** | **V1** | **V2** | **V3** | **V4** | **V5** | **V6** | **Mean** | **%RSD** |
| | | | 15 | 72 | 72 | 31 | 73 | 29 | 24 | **50** | 48.8% |
| | | | 45 | 93 | 93 | 91 | 88 | 91 | 92 | **91** | 2.0% |
| T=2 Month | 40 °C/75% RH | 1.1.6.326 | **Time (min)** | **V1** | **V2** | **V3** | **V4** | **V5** | **V6** | **Mean** | **%RSD** |
| | | | 15 | 47 | 23 | 23 | 59 | 75 | 26 | **42** | 51.8% |
| | | | 45 | 97 | 96 | 95 | 96 | 92 | 96 | 95 | 2.1% |
| T=3 Month | 40 °C/75% RH | OTBA-THPLC-0293 | **Time (min)** | **V1** | **V2** | **V3** | **V4** | **V5** | **V6** | **Mean** | **%RSD** |
| | | | 10 | 4 | 13 | 9 | 7 | 9 | 19 | **10** | 54.7% |
| | | | 20 | 75 | 55 | 56 | 80 | 84 | 95 | **74** | 21.6% |
| | | | 35 | 88 | 95 | 96 | 91 | 93 | 98 | **93** | 3.7% |
| | | | 45 | 91 | 97 | 98 | 94 | 96 | 98 | **96** | 2.8% |

**Table 8 - Naproxen Mini Softgel Formulation - Dissolution Results - Batch 2**

| **Time point** | **Condition** | **Method** | **Dissolution** | | | | | | | | |
|---|---|---|---|---|---|---|---|---|---|---|---|
| T=0 | N/A | OTBA-THPLC-0293 | **Time (min)** | **V1** | **V2** | **V3** | **V4** | **V5** | **V6** | **Mean** | **%RSD** |
| | | | 10 | 23 | 46 | 25 | 33 | 27 | 20 | **29** | 32.8% |
| | | | 20 | 74 | 88 | 86 | 88 | 84 | 87 | **85** | 6.3% |
| | | | 35 | 94 | 94 | 97 | 97 | 99 | 99 | **97** | 2.4% |
| | | | 45 | 98 | 97 | 100 | 100 | 100 | 101 | **99** | 1.5% |
| T=1 Month | 40 °C/75% RH | 1.1.6.326 | **Time (min)** | **V1** | **V2** | **V3** | **V4** | **V5** | **V6** | **Mean** | **%RSD** |
| | | | 15 | 66 | 37 | 69 | 82 | 65 | 71 | 65 | 23.1% |
| | | | 45 | 100 | 99 | 99 | 99 | 92 | 98 | 98 | 2.8% |
| T=2 Month | 40 °C/75% RH | 1.1.6.326 | **Time (min)** | **V1** | **V2** | **V3** | **V4** | **V5** | **V6** | **Mean** | **%RSD** |
| | | | 15 | 69 | 77 | 69 | 75 | 62 | 42 | **66** | 19.3% |
| | | | 45 | 94 | 95 | 94 | 98 | 99 | 98 | **96** | 2.4% |
| T=3 Month | 40 °C/75% RH | OTBA-THPLC-0293 | **Time (min)** | **V1** | **V2** | **V3** | **V4** | **V5** | **V6** | **Mean** | **%RSD** |
| | | | 10 | 14 | 14 | 12 | 14 | 12 | 41 | **18** | 63.2% |
| | | | 20 | 76 | 77 | 64 | 76 | 67 | 85 | **74** | 9.8% |
| | | | 35 | 99 | 99 | 95 | 99 | 97 | 94 | **97** | 2.5% |
| | | | 45 | 100 | 101 | 99 | 101 | 99 | 97 | **99** | 1.4% |
| T=6 Month | 30 °C / 65% RH | 1.1.6.481 | **Time (min)** | **V1** | **V2** | **V3** | **V4** | **V5** | **V6** | **Mean** | **%RSD** |
| | | | 10 | 37 | 63 | 85 | 48 | 21 | 96 | **58** | 49.3% |
| | | | 15 | 74 | 87 | 98 | 85 | 71 | 101 | **86** | 14.1% |
| | | | 20 | 83 | 94 | 101 | 94 | 86 | 101 | **93** | 8.0% |
| | | | 30 | 91 | 99 | 102 | 100 | 94 | 102 | **98** | 4.5% |
| | | | 45 | 99 | 102 | 102 | 102 | 99 | 102 | **101** | 1.4% |
| T=6 Month | 40 °C / 75% | 1.1.6.481 | **Time (min)** | **V1** | **V2** | **V3** | **V4** | **V5** | **V6** | **Mean** | **%RSD** |
| | | | 10 | 12 | 12 | 8 | 34 | 13 | 7 | **14** | 68.6% |
| | | | 15 | 34 | 44 | 26 | 74 | 60 | 59 | **49** | 36.8% |
| | | | 20 | 74 | 66 | 59 | 90 | 75 | 79 | **74** | 14.5% |
| | | | 30 | 92 | 90 | 87 | 95 | 81 | 87 | **89** | 5.5% |
| | | | 45 | 98 | 94 | 92 | 96 | 86 | 92 | **93** | 4.3% |
| T=9 Month | 30 °C / 65% RH | 1.1.6.481 | **Time (min)** | **V1** | **V2** | **V3** | **V4** | **V5** | **V6** | **Mean** | **%RSD** |
| | | | 10 | 14 | 30 | 15 | 17 | 10 | 17 | **17** | 40.0% |
| | | | 15 | 69 | 79 | 65 | 62 | 66 | 63 | **67** | 9.2% |
| | | | 20 | 93 | 88 | 91 | 83 | 78 | 81 | **86** | 6.9% |
| | | | 30 | 97 | 94 | 99 | 94 | 87 | 95 | **94** | 4.4% |
| | | | 45 | 100 | 99 | 101 | 99 | 94 | 100 | **99** | 2.7% |

In comparison, a comparative formulation of a commercial product, including 200 mg related active agents, displayed the dissolution results depicted in Table 9 below. The comparative formulation was purchased and tested directly from the commercial bottle.

**Table 9 - Comparative Naproxen Formulation - Dissolution Results**

| **Method** | **Dissolution** | | | | | | | | |
|---|---|---|---|---|---|---|---|---|---|
| OTBA-THPLC-0293 | **Time (min)** | **V1** | **V2** | **V3** | **V4** | **V5** | **V6** | **Mean** | **%RSD** |
| | 10 | 19 | 58 | 56 | 25 | 60 | 37 | **42** | 42.5% |
| | 20 | 89 | 90 | 92 | 77 | 79 | 90 | **86** | 7.3% |
| | 35 | 98 | 95 | 97 | 88 | 90 | 94 | **94** | 4.0% |
| | 45 | 100 | 98 | 99 | 93 | 94 | 98 | **97** | 2.9% |

The stability study results and dissolution results for both batches of naproxen mini softgel formulations according to embodiments described herein (and particularly of the second batch) were comparable to the stability study results and dissolution results of the comparative naproxen formulation.

Analytical method 1.1.6.211 was used to determine the amount of naproxen related substances in a naproxen 200 mg soft gelatin capsules, by HPLC. The column used was YMC-Pack Pro C18 RS, 3 µm, 150 x 4.6 mm at 30 °C. Detection was at 230 nm. The sample was kept at ambient temperature and a volume of 10.0 µL was injected for analysis by HPLC.

The stock sample solution for testing using analytical method 1.1.6.211 was prepared as follows:
- Five capsules of naproxen were placed into a 500 mL volumetric flask,
- 200 mL of diluent were added to the flask
- The flask was heated in a 50 °C water bath for at least 1 hour with occasional rapid swirling until no gel stuck to the bottom of the flask and the capsules were completely dissolved
- The flask was removed from the water bath
- 200 mL methanol was added and the solution was shaken well
- The flask was sonicated for five minutes
- The solution was allowed to come to room temperature where it was made up to volume with methanol and mixed well
- The solution was allowed to stand at room temperature for 10 minutes to settle undissolved material
- At each time point, 5 mL of the stock sample solution supernatant was placed into a 100 mL volumetric flask and made up to volume with the diluent to make the sample that was analyzed

The diluent was 2.62 g sodium phosphate monobasic and 11.50 sodium phosphate dibasic in 1000 ml water, mobile phase A was 0.1% H₃PO₄, and mobile phase B was acetonitrile. The chromatographic conditions that were used were in accordance with Table 10 below:

| Table 10 - Gradient Table for HPLC Method used in Analytical Method 1.1.6.211 | | | |
|---|---|---|---|
| Time (min) | Flow (mL/min) | % Mobile Phase A | % Mobile Phase B |
| Initial | 1.0 | 95 | 5 |
| 5 | 1.0 | 95 | 5 |
| 20 | 1.0 | 60 | 40 |
| 26 | 1.0 | 60 | 40 |
| 40 | 1.0 | 10 | 90 |
| 40.1 | 1.0 | 95 | 5 |
| 45 | 1.0 | 95 | 5 |

For simplicity of explanation, the embodiments of the methods of this disclosure are depicted and described as a series of acts. However, acts in accordance with this disclosure can occur in various orders and/or concurrently, and with other acts not presented and described herein. Furthermore, not all illustrated acts may be required to implement the methods in accordance with the disclosed subject matter. In addition, those skilled in the art will understand and appreciate that the methods could alternatively be represented as a series of interrelated states via a state diagram or events.

The words "example" or "exemplary" are used herein to mean serving as an example, instance, or illustration. Any aspect or design described herein as "example" or "exemplary" is not necessarily to be construed as preferred or advantageous over other aspects or designs. Rather, use of the words "example" or "exemplary" is intended to present concepts in a concrete fashion. As used in this application, the term "or" is intended to mean an inclusive "or" rather than an exclusive "or". That is, unless specified otherwise, or clear from context, "X includes A or B" is intended to mean any of the natural inclusive permutations. That is, if X includes A; X includes B; or X includes both A and B, then "X includes A or B" is satisfied under any of the foregoing instances. Reference throughout this specification to "an embodiment", "certain embodiments", or "one embodiment" means that a particular feature, structure, or characteristic described in connection with the embodiment is included in at least one embodiment. Thus, the appearances of the phrase "an embodiment", "certain embodiments", or "one embodiment" in various places throughout this specification are not necessarily all referring to the same embodiment.

## Claims

1. A pharmaceutical composition comprising a softgel and a fill composition, the fill composition comprising a reaction product of naproxen free acid and potassium hydroxide, wherein a molar ratio of the potassium hydroxide to naproxen free acid is less than 1 (+/-5%), and wherein the softgel has a size 8 to 14, wherein the potassium salt of naproxen is present in the fill composition at a concentration of from about 65 wt% to about 75 wt% based on a total combined weight of the naproxen free acid and the potassium salt of naproxen.

2. The pharmaceutical composition of claim 1, wherein the fill composition has a weight of from 200 mg (+/-5%) to 800 mg (+/-5%), preferably from 600 mg (+/-5%) to 700 mg (+/-5%).

3. The pharmaceutical composition of claim 1 or 2, wherein the softgel has a size of from 10 to 12, or additionally or alternatively, the softgel has an oblong shape or an oval shape.

4. The pharmaceutical composition of any one of the preceding claims, wherein the potassium salt of naproxen is present in the fill composition at a concentration of from 65 wt% to 70 wt%, based on a total combined weight of the naproxen free acid and the potassium salt of naproxen.

5. The pharmaceutical composition of any one of the preceding claims, wherein the fill composition has a moisture content of up to 15%, up to 12%, up to 10%, up to 9%, up to 8%, up to 7%, up to 6%, up to 5%, up to 4%, up to 3%, up to 2%, up to 1%, or 0%, or alternatively, having a hardness of 2 N to 20 N, of 4 N to 15 N, of 6 N to 13 N, or of 8 N to 12 N (+/-5%).

6. A method of preparing a pharmaceutical composition according to any one of the preceding claims 1 to 5, the method comprising incorporating naproxen free acid and potassium hydroxide in a mini softgel capsule.

7. The pharmaceutical composition according to any one of claims 1 to 5 for use as a medicament.

## Patentansprüche

1. Pharmazeutische Zusammensetzung, umfassend eine Weichkapsel und eine Füllzusammensetzung, wobei die Füllzusammensetzung ein Reaktionsprodukt aus freier Naproxensäure und Kaliumhydroxid umfasst, wobei das Molverhältnis von Kaliumhydroxid zu freier Naproxensäure weniger als 1 (+/-5%) beträgt, und wobei die Weichkapsel eine Größe von 8 bis 14 hat, wobei das Kaliumsalz von Naproxen in der Füllzusammensetzung in einer Konzentration von etwa 65 Gew.-% bis etwa 75 Gew.-%, bezogen auf das kombinierte Gesamtgewicht der naproxenfreien Säure und des Kaliumsalzes von Naproxen, vorhanden ist.

2. Pharmazeutische Zusammensetzung nach Anspruch 1, wobei die Füllzusammensetzung ein Gewicht von 200 mg (+/-5%) bis 800 mg (+/-5%), vorzugsweise von 600 mg (+/-5%) bis 700 mg (+/-5%), aufweist.

3. Pharmazeutische Zusammensetzung nach Anspruch 1 oder 2, wobei die Weichkapsel eine Größe von 10 bis 12 hat, oder zusätzlich oder alternativ die Weichkapsel eine längliche Form oder eine ovale Form hat.

4. Pharmazeutische Zusammensetzung nach einem der vorhergehenden Ansprüche, wobei das Kaliumsalz von Naproxen in der Füllzusammensetzung in einer Konzentration von 65 Gew.-% bis 70 Gew.-%, bezogen auf das kombinierte Gesamtgewicht der freien Säure von Naproxen und des Kaliumsalzes von Naproxen, vorhanden ist.

5. Pharmazeutische Zusammensetzung nach einem der vorhergehenden Ansprüche, wobei die Füllzusammensetzung einen Feuchtigkeitsgehalt von bis zu 15 %, bis zu 12 %, bis zu 10 %, bis zu 9 %, bis zu 8 %, bis zu 7 %, bis zu 6 %, bis zu 5 %, bis zu 4 %, bis zu 3 %, bis zu 2 %, bis zu 1 % oder 0 % aufweist oder alternativ eine Härte von 2 N bis 20 N, von 4 N bis 15 N, von 6 N bis 13 N oder von 8 N bis 12 N (+/-5 %) aufweist.

6. Verfahren zur Herstellung einer pharmazeutischen Zusammensetzung nach einem der vorhergehenden Ansprüche 1 bis 5, wobei das Verfahren das Einbringen von naproxenfreier Säure und Kaliumhydroxid in eine Mini-Softgel-Kapsel umfasst.

7. Pharmazeutische Zusammensetzung nach einem der Ansprüche 1 bis 5 zur Verwendung als Arzneimittel.

## Revendications

1. Composition pharmaceutique comprenant une enveloppe molle et une composition de remplissage, la composition de remplissage comprenant un produit réactionnel d'acide libre de naproxène et d'hydroxyde de potassium, dans laquelle un rapport molaire de l'hydroxyde de potassium à l'acide libre de naproxène est inférieur à 1 (+/-5 %), et dans laquelle l'enveloppe molle a une taille de 8 à 14, dans laquelle le sel de potassium de naproxène est présent dans la composition de remplissage en une concentration d'environ 65 % en poids à environ 75 % en poids sur la base d'un poids total combiné de l'acide libre de naproxène et du sel de potassium de naproxène.

2. Composition pharmaceutique selon la revendication 1, dans laquelle la composition de remplissage a un poids de 200 mg (+/-5 %) à 800 mg (+/-5 %), de préférence de 600 mg (+/-5 %) à 700 mg (+/-5 %).

3. Composition pharmaceutique selon la revendication 1 ou 2, dans laquelle l'enveloppe molle a une taille de 10 à 12, ou en complément ou en variante, l'enveloppe molle a une forme oblongue ou une forme ovale.

4. Composition pharmaceutique selon l'une des revendications précédentes, dans laquelle le sel de potassium de naproxène est présent dans la composition de remplissage en une concentration de 65 % en poids à 70 % en poids, sur la base d'un poids total combiné de l'acide libre de naproxène et du sel de potassium de naproxène.

5. Composition pharmaceutique selon l'une des revendications précédentes, dans laquelle la composition de remplissage a une teneur en humidité allant jusqu'à 15 %, jusqu'à 12 %, jusqu'à 10 %, jusqu'à 9 %, jusqu'à 8 %, jusqu'à 7 %, jusqu'à 6 %, jusqu'à 5 %, jusqu'à 4 %, jusqu'à 3 %, jusqu'à 2 %, jusqu'à 1 %, ou 0 %, ou en variante, ayant une dureté de 2 N à 20 N, de 4 N à 15 N, de 6 N à 13 N, ou de 8 N à 12 N (+/-5 %).

6. Procédé de préparation d'une composition pharmaceutique selon l'une des revendications 1 à 5 précédentes, le procédé comprenant l'incorporation d'acide libre de naproxène et d'hydroxyde de potassium dans une mini capsule à enveloppe molle.

7. Composition pharmaceutique selon l'une des revendications 1 à 5 pour une utilisation en tant que médicament.
